# EUROPEAN PATENT APPLICATION

(11) **EP 3 130 333 A1**
(43) Date of publication of application: **15.02.2017**
(21) Application number: 15776327.7
(22) Date of filing: 07.04.2015
(51) Int. Cl.: A61K 9/16, A61K 9/107

(54) **METHOD FOR PREPARING POLYMERIC MICROSPHERES CONTAINING DRUG**

(30) Priority: 07.04.2014 KR 20140041299
(71) Applicant: SK Chemicals Co., Ltd., Seongnam-si, Gyeonggi-do 463-400 (KR)
(72) Inventor: KIM, Hong Kee, Gunpo-si Gyeonggi-do 435-704 (KR); SHIN, Ho Chul, Seongnam-si Gyeonggi-do 463-748 (KR); LEE, Yoon-Jung, Yongin-si Gyeonggi-do 446-951 (KR); HONG, Seok Hyun, Suwon-si Gyeonggi-do 443-811 (KR); LEE, Kyu Ho, Seoul 137-838 (KR); KIM, Hun-Teak, Seoul 137-951 (KR); KWAK, Yong-Kyu, Seoul 121-824 (KR)
(74) Representative: Isarpatent
(86) International application number: PCT/KR2015/003455
(87) International publication number: WO 2015/156572

(57) **Abstract**

The present invention relates to a method for preparing polymeric microspheres to improve the drug encapsulating efficiency and, more specifically, to a method for preparing polymeric microspheres, the method comprising the steps of: preparing an internal water phase (w1) and an oil phase (0); mixing the internal water phase and the oil phase to prepare a W1/0 emulsion, followed by cooling; mixing the W1/0 emulsion with an external water phase (W2) to prepare a W1/0/W2 emulsion; and drying the prepared W1/0/W2 emulsion, followed by collection. The method of the present invention can increase the drug encapsulating efficiency and manufacture polymeric microspheres having high drug contents through a procedure of maintaining the step of solidifying the oil phase at a particular temperature for a particular period of time, and thus the method of the present invention is effective in the preparation of sustained release-type drugs.

## Description

### Technical Field

This application claims priority from and the benefit of Korean Patent Application No. 10-2014-0041299 filed on 07 April 2014, which is hereby incorporated herein by reference in its entity.

The present invention relates to a method for preparing polymeric microspheres having an increased efficiency of drug encapsulation and, more specifically, to a method for preparing polymeric microspheres, the method comprising the steps of: preparing an internal water phase (W1) and an oil phase (O); mixing the internal water phase and the oil phase to prepare a W1/O emulsion and cooling the W1/O emulsion; mixing the W1/O emulsion with an external water phase (W2) to prepare a W1/O/W2 emulsion; and drying and recovering the prepared W1/O/W2 emulsion

### Background Art

Controlled-release preparations, compared with ordinary rapid-release preparations, continuously release drugs in the body for a predetermined time, and thus can maintain the effective blood levels of contained drugs for a long period of time. Therefore, the controlled-release preparations can decrease the fluctuation of blood levels caused by frequent administrations of ordinary preparations, with the concomitant reduction of the effects accompanying the fluctuating blood levels, and further can decrease the frequency of administration, thereby improving drug compliance.

For the development of such controlled-release preparations, a variety of experiments on drug delivery using biodegradable polymeric microspheres have recently been conducted. The biodegradable polymeric microsphere drug delivery system can control the dose of drugs within a fixed quantity for a desired period of time, reduce side effects, and increase the effects of drugs.

In the development of drug delivery systems using polymeric microspheres, one of the important matters is to increase the drug encapsulation efficiency in view of productivity.

According to the prior art, USP 4,954,298, in the preparation of controlled-release preparations on the basis of polymeric microspheres composed of internal water phase (W1)/oil phase (0)/external water phase (W2), the drug encapsulation efficiency is improved by cooling the internal water phase (W1)/oil phase (O) emulsion to increase the viscosity thereof.

However, there is a need to improve an additional process for increasing the drug encapsulation efficiency.

### Detailed Description of the Invention

### Technical Problem

The present inventors, while researching methods for increasing the drug encapsulation efficiency in the preparation of W1/O/W2 type drug-containing polymeric microspheres, established that the drug encapsulation efficiency is significantly increased when W1/O/W2 is prepared by mixing a W1/O emulsion and W2 in the conditions in which a particular temperature or lower is maintained for a predetermined period of time, and then completed the present invention.

Therefore, the present invention has been made in view of the above-mentioned problems, and an aspect of the present invention relates to a method for preparing polymeric microspheres, the method comprising the steps of: (a) preparing an internal water phase (W1) containing a drug, a thickener, and an aqueous solvent, and preparing an oil phase (O) containing a polymer and a fat-soluble solvent; (b) mixing the internal water phase and oil phase prepared in step (a) to prepare a W1/O emulsion, and cooling the prepared W1/O emulsion to increase viscosity of the W1/O emulsion; (c) dispersing the W1/O emulsion, of which the viscosity is increased in step (b), in an external water phase (W2), which contains a water-soluble solution and of which the temperature is maintained at 18°C or lower, followed by mixing while the temperature is maintained at 18°C or lower for 10 minutes or longer, thereby preparing a W1/O/W2 emulsion; and (d) drying and recovering the W1/O/W2 emulsion prepared in step (c).

Another aspect of the present invention is to provide polymeric microspheres prepared by the preparation method above.

### Technical Solution

In accordance with an aspect of the present invention, there is provided a method for preparing polymeric microspheres, the method comprising the steps of: (a) preparing an internal water phase (W1) containing a drug, a thickener, and an aqueous solvent, and preparing an oil phase (O) containing a polymer and a fat-soluble solvent; (b) mixing the internal water phase and the oil phase prepared in step (a) to prepare a W1/O emulsion, and cooling the prepared W1/O emulsion to increase viscosity of the W1/O emulsion; (c) dispersing the W1/O emulsion, of which the viscosity is increased in step (b), in an external water phase (W2), which contains a water-soluble solution and of which the temperature is maintained at 18°C or lower, followed by mixing while the temperature is maintained at 18°C or lower for 10 minutes or longer, thereby preparing a W1/O/W2 emulsion; and (d) drying and recovering the W1/O/W2 emulsion prepared in step (c).

In accordance with another aspect of the present invention, there are provided polymeric microspheres prepared by the preparation method above.

Hereafter, the present invention will be described in detail.

The present invention provides a method for preparing polymeric microspheres, the method comprising the steps of:
(a) preparing an internal water phase (W1) containing a drug, a thickener, and an aqueous solvent, and preparing an oil phase (O) containing a polymer and a fat-soluble solvent;
(b) mixing the internal water phase and the oil phase prepared in step (a) to prepare a W1/O emulsion, and cooling the prepared W1/O emulsion to increase viscosity of the W1/O emulsion;
(c) dispersing the W1/O emulsion, of which the viscosity is increased in step (b), in an external water phase (W2), which contains a water-soluble solution and of which the temperature is maintained at 18°C or lower, followed by mixing while the temperature is maintained at 18°C or lower for 10 minutes or longer, thereby preparing a W1/O/W2 emulsion; and
(d) drying and recovering the W1/O/W2 emulsion prepared in step (c).

Hereinafter, the preparation method of the present invention will be described by each steps.

### (a) Step for preparing an internal water phase (W1) containing a drug, a thickener, and an aqueous solvent, and preparing an oil phase (O) containing a polymer and a fat-soluble solvent

In step (a), an internal water phase and an oil phase for preparing a W1/O emulsion is prepared. The internal water phase of the present invention is characterized by containing a drug, a thickener, and an aqueous solvent, and the oil phase is characterized by containing a polymer and a fat-soluble solvent.

The drug as described above refers to a medicinal agent, a physiological agent, a bioactive compound, or a mixture thereof, which is useful for the diagnosis, treatment, alleviation, therapy, or prevention of diseases, or is useful for other medical purposes. The drug of the present invention includes chemical compounds, peptides, proteins, antibodies, nucleic acids, or salts thereof. Preferably, the drug of the present invention may be a water-soluble drug.

The drug of the present invention may be, more preferably, a peptide having bioactivity, and most preferably, leuprorelin acetate.

Leuprorelin acetate is a hormone agent classified as a luteinizing hormone-releasing hormone (LHRH) agonist, and also called leuprolide, leuprorelin acetate, or leuprolide acetate. The leuprorelin acetate causes a lack of testosterone or estrogen in testosterone or estrogen-dependent cancer cells, thereby reducing or contracting tumor sizes. The leuprorelin acetate is mainly used for the treatment of breast cancer, prostate cancer responding to hormones, endometrial cancer, uterine myoma, and pubertas praecox, but may also be used for the purpose of the treatment of other diseases.

The thickener increases the viscosity of the internal water phase, and thus improves the effect of increasing viscosity when the W1/O emulsion is prepared and cooled. Examples of the thickener may include polymeric compounds (such as casein, gelatin, and collagen), carbohydrates (such as cellulose, dextrin, and agar), and natural rubbers (such as xanthan gum). The thickener may preferably be gelatin.

The aqueous solvent refers to a solvent capable of dissolving the drug of the present invention therein, and any aqueous solvent that is used in the solution of a drug may be used without limitation. Examples of the aqueous solvent of the present invention may include, but are not limited to, water, C1-C4 lower alcohols (e.g., methanol, ethanol, propanol, etc.), acetonitrile, acetone, and tetrahydrofuran. Preferably, the aqueous solvent of the present invention may be ethanol, methanol, water, or a mixture thereof. More preferably, the aqueous solvent of the present invention may be water.

The weight ratio of the aqueous solvent, the drug, and the thickener constituting the internal water phase (W1) may vary depending on the kind of the aqueous solvent and the kind of the drug. For example, the aqueous solvent, the drug, and the thickener may be mixed at a weight ratio of 1 : 0.01-2 : 0.01-0.5, and may preferably be mixed at a ratio of 0.01-1 parts by weight of the drug, 0.05-0.2 parts by weight of the thickener on the basis of 1 part by weight of the aqueous solvent.

The internal water phase (W1) may be prepared by sequentially or simultaneously mixing the aqueous solvent, the drug, and the thickener, and may preferably be mixed in the conditions under which the internal water phase is warmed. The warming condition may be preferably 30-80°C, and more preferably 50-70°C.

The polymer refers to a polymeric compound constituting an outer wall of the polymeric microsphere. As the polymer of the present invention, any polymeric compound that is used to prepare the polymeric microspheres and is known in the art may be used without limitation. Examples of the polymeric compound may include, but are not limited to, as polyesters of hydroxy fatty acid, copolymers of poly(lactic acid) and poly(glycolic acid), polymers of only poly(lactic acid) or polylactide, polylactic-co-glycolic acid, polylactide-co-glycolide (PLGA), polyphosphazenes, polyiminocarbonates, polyphosphoesters, polyanhydrides, polyorthoesters, copolymers of lactic acid and caprolactone, polycaprolactones, polyhydroxyvalerates, polyhydroxybutyrates, polyamino acids, copolymers of lactic acid and amino acids, and mixtures thereof. Preferably, the polymeric compound may be a copolymer of poly(lactic acid) and poly(glycolic acid). The weight-average molecular weight of the polymer used in the preparation method of the present invention is not particularly limited, and may be generally 2,000-800,000, preferably 5,000-100,000, and more preferably 10,000-50,000.

The molar ratio of lactic acid and glycolic acid in the copolymer of poly(lactic acid) and poly(glycolic acid) may be 0-100 : 0-100, preferably 50-90 : 10-50 (e.g., 50:50, 65:35, 75:25, 85:15, 90: 10), and more preferably 70-80 : 20-30 (e.g., 75:25) .

The fat-soluble solvent is used for dissolving a high-molecular weight polymer, and any fat-soluble solvent that is used to prepare polymeric microspheres for controlled-release preparations may be generally used without limitation. Examples of the fat-soluble solvent of the present invention may include halogenated hydrocarbons (such as, dichloromethane, chloroform, dichloroethane, trichloroethane, and carbon tetrachloride), ethers (such as ethyl ether and isopropyl ether), fatty esters (ethyl acetate and butyl acetate), and aromatic hydrocarbons (such as benzene, toluene, and xylene). The fat-soluble solvent may be preferably a halogenated hydrocarbon, and more preferably dichloromethane.

The amount of the fat-soluble solvent is not particularly limited so long as the polymer can be dissolved in the fat-soluble solvent. The amount of the fat-soluble solvent varies depending on the kinds of the polymer and the fat-soluble solvent, but may be for example 0.1-fold to 10-fold, preferably 0.5-fold to 4-fold, and more preferably 1-fold to 2-fold, of the weight of the polymer.

The oil phase may be prepared by mixing the polymer in the fat-soluble solvent, and the mixing may be carried out using a stirrer or mixer, or a shaker (vortexer).

### (b) Step for mixing the internal water phase and the oil phase prepared in step (a) to prepare a W1/O emulsion, and cooling the prepared W1/O emulsion to increase the viscosity of the W1/O emulsion

In step (b), the internal water phase and the oil phase prepared in step (a) are mixed to prepare a W1/O emulsion, and then the W1/O emulsion is cooled to increase the viscosity of the W1/O emulsion.

The W1/O emulsion is prepared by mixing the internal water phase and the oil phase, and the mixing may be conducted by a known method. The mixing may be conducted by, for example, a discontinuous shaking method, a stirring method using a propeller type stirrer or a turbine type stirrer, a colloid milling method, a homogenizer method, or an ultrasonic method, and preferably a homogenizer method. The W1/O emulsion of the present invention may preferably be prepared by injecting the internal water phase in the oil phase and homogenizing the mixture using a homogenizer.

The mixing ratio of the internal water phase and the oil phase may vary depending on the kinds of the polymer, the drug, the aqueous solvent, and the fat-soluble solvent. The internal water phase and the oil phase may be mixed at a ratio of, for example, 1-100 parts by weight of the oil phase relative to 1 part by weight of the internal water phase, preferably 1-20 parts by weight of the oil phase relative to 1 part by weight of the internal water phase, and more preferably 5-10 parts by weight of the oil phase relative to 1 part by weight of the internal water phase.

The prepared W1/O emulsion is cooled to increase the viscosity thereof.

The increased viscosity may partially improve the efficiency of drug encapsulation. However, the effect of increasing the encapsulation efficiency solely due to the increased viscosity of the W1/O emulsion has a limitation in the increment thereof, so sufficient encapsulation efficiency cannot be attained in the preparation of the controlled-release preparation. It is necessary to control the temperature of the external water phase (W2) and the time period for maintaining the temperature.

The cooling temperature is not particularly limited so long as the temperature is sufficient to increase the viscosity of the W1/O emulsion. The cooling temperature may preferably be 18°C or lower.

### (c) Step for dispersing the W1/O emulsion, of which viscosity is increased in step (b), in an external water phase (W2), which contains a water-soluble solution and of which temperature is maintained at 18°C or lower, followed by mixing while temperature is maintained at 18°C or lower for 10 minutes or more, thereby preparing a W1/O/W2 emulsion

In step (c), the W1/O emulsion with increased viscosity is dispersed in an external water phase (W2) to prepare a W1/O/W2 emulsion.

The external water phase (W2) of the present invention is characterized by containing a water-soluble solution.

The water-soluble solution refers to a solution in which an emulsifier (emulsion stabilizer) is dissolved in an aqueous solvent.

Examples of the aqueous solvent may include water, C1-C4 lower alcohols (such as methanol, ethanol, and propanol), acetonitrile, acetone tetrahydrofurane, and mixtures thereof. Preferably, the aqueous solvent may be ethanol, methanol, water, or a mixture thereof. More preferably, the aqueous solvent may be water.

The external water phase (W2) of the present invention may further contain, in addition to the emulsifier, a suitable buffer for adjusting the pH of the external water phase, and an osmoregulator (e.g., mannitol) for regulating osmotic pressure. The encapsulation efficiency and stability of drugs can be increased by the buffer or the osmoregulator.

The emulsifier may be a known emulsifier that stably forms an emulsion. Examples of the emulsifier may include anionic surfactants (such as gelatin, sodium oleate, sodium stearate, sodium lauryl sulfate, and sodium dodecyl sulfate (SDS)), non-anionic surfactants (such as polyoxyethylene sorbitan fatty esters (polysorbates), for instance, Tween 80 and Tween 60, polyoxyethylene castor oil derivatives), polyvinyl alcohol, carboxymethyl cellulose, lecithin, hyaluronic acid, and mixtures thereof. Preferably, the emulsifier may be polyvinyl alcohol (PVA).

The step for preparing the W1/O/W2 emulsion of the present invention is characterized in that the W1/O emulsion with increased viscosity is dispersed in a state in which the temperature of the external water phase (W2) is maintained at 18°C or lower, and even in the dispersion step, the temperature is maintained at 18°C or lower for at least 10 minutes.

The drug encapsulation efficiency is greatly increased by maintaining the temperature at 18°C or lower for at least 10 minutes in the step for dispersing the W1/O emulsion in the external water phase. These findings are first disclosed in the present invention.

In order to maintain the temperature in the dispersion step at 18°C or lower, the temperature of the external water phase (W2) is preferably maintained at 18°C, and more preferably, may be in a range of 10°C to 18°C.

The dispersing and mixing of the W1/O emulsion with increased viscosity in the external water phase (W2) may be carried out by known mixing methods, which are described as in the foregoing method of mixing the internal water phase and the oil phase in the preparation of the W1/O emulsion.

The temperature for dispersing and mixing the W1/O emulsion with increased viscosity in the external water phase (W2) is preferably 18°C or lower, more preferably being in a range of 10°C to 18°C, and most preferably being in a range of 15°C to 18°C.

In addition, the temperature is preferably maintained for at least 10 minutes. The drug encapsulation efficiency is sharply decreased if the temperature is maintained for less than 10 minutes. The maintenance period of time may be more preferably in a range of 10 minutes to 120 minutes, and most preferably in a range of 30 minutes to 90 minutes. A maintenance period of time of 120 minutes or more may be inefficient since the increment in encapsulation efficiency through the increased maintenance period of time is not great depending on the composition of the polymeric microspheres.

In Examples and Comparative Examples of the present invention, the W1/O emulsion was prepared while various temperatures were maintained for various periods of time, and the drug encapsulation efficiency and the drug content were measured.

As a result, it was verified that the drug encapsulation efficiency was very low when the initial temperature was 20°C and 25°C, respectively (Comparative Examples 1 and 2), while the drug encapsulation efficiency was low, about 20%, even when the initial temperature of 18°C was not maintained for 10 minutes or more (Comparative Example 3).

On the contrary, it was found that, when the W1/10 emulsion with an increased viscosity was dispersed in the external water phase (W2), the temperature was maintained at 18°C for 10 minutes and then gently raised, leading to a remarkable improvement in drug encapsulation efficiency (Example 1). It was also found that in case where the temperature was maintained for 30 minutes, a more remarkable improvement in drug encapsulation efficiency was obtained (Examples 2 to 4).

### (d) Step for drying and recovering the W1/O/W2 emulsion prepared in step (c)

In step (d), the prepared W1/O/W2 emulsion is dried to prepare polymeric microspheres, which is then recovered.

In order to remove the solvent from the prepared polymeric microspheres, the drying may be carried out by an ordinary method that is generally used. For example, the prepared polymeric microspheres may be dried by simple stirring, heating, addition of nitrogen gas or the like, stirring under a reduced pressure condition, or evaporation of the solvent under a controlled vacuum condition. Preferably, the drying may be carried out by an in-water drying method of removing a solvent through stirring under a reduced pressure condition.

The recovering as described above refers to separating and obtaining the polymeric microspheres from the water phase. The recovering may be carried out by an ordinary method of separating solids from a liquid phase, such as centrifugation or filtration.

The polymeric microspheres prepared in step (d) may further pass through a washing step for re-dispersing and mixing the polymeric microspheres in distilled water or the like and then re-recovering the polymeric microspheres. Through the washing step above, the drug residue on surfaces of the microspheres and the emulsion stabilizer contained in the external water phase (W2) may be removed.

Furthermore, the polymeric microspheres prepared by the preparation method of the present invention are solidified by a freeze-drying method or the like, thereby preparing more stable polymeric microspheres. More preferably, the prepared polymeric microspheres are suspended in a suitable solution, such as water for injection, and the suspension was mixed with a known excipient (e.g., sugar, such as mannitol), a dispersant, and the like, followed by freeze-drying, so that the polymeric microspheres can be solidified.

According to the preparation method of the present invention, the polymeric microspheres can be prepared with high efficiency of drug encapsulation, thereby reducing the loss of drug, so controlled-release preparations can be economically produced. In addition, polymeric microspheres with an improved drug content can be prepared compared with conventional methods.

Therefore, the present invention provides polymeric microspheres prepared by the method of the present invention.

Compared with polymeric microspheres prepared by conventional methods, the polymeric microspheres of the present invention can be more economically produced through high efficiency of drug encapsulation, together with a high drug content.

The polymeric microspheres prepared by the method of the present invention can be used for the treatment of a target disease according to the kind of the drug contained in the polymeric microspheres, and therefore, the present invention provides a pharmaceutical composition for treating a disease, containing the polymeric microspheres of the present invention as an active ingredient.

The disease as described above is a disease that can be treated by the drug contained in the polymeric microspheres of the present invention. Examples of the disease may include prostate cancer, endometrial cancer, uterine myoma, and pubertas praecox.

The pharmaceutical composition according to the present invention may contain a pharmaceutically effective amount of drug-containing polymeric microspheres alone or may further contain at least one pharmaceutically acceptable carrier. As used herein, the term "pharmaceutically effective amount" refers to an amount required to exhibit a higher response compared with a negative control, and preferably an amount sufficient to treat or prevent a disease. The pharmaceutically effective amount of the polymeric microspheres according to the present invention is 0.001-1000 mg/day/kg body weight, and preferably 0.005-1 mg/day/kg body weight. However, the pharmaceutically effective amount may vary depending on disease and severity thereof, age, body weight, physical conditions and sex of a patient, administration route, treatment period, or other various factors.

As used herein, the term "pharmaceutically acceptable" composition refers to a non-toxic composition that is physiologically acceptable, does not inhibit an action of an active ingredient when administered to humans, and does not ordinarily cause an allergic reaction or similar reactions, such as gastroenteric troubles and dizziness. The composition of the present invention may be variously formulated, together with the pharmaceutically acceptable carrier, depending on a route of administration, by methods known in the art. The route of administration may include, but is not limited to, oral administration or parenteral administration. Examples of the route of parenteral administration include several routes, such as transdermal, intranasal, intraperitoneal, intramuscular, subcutaneous, and intravenous routes.

As for the parenteral administration, the pharmaceutical composition of the present invention may be formulated in a dosage form of an injection, a transdermal administration preparation, and a nasal inhalant, together with a suitable parenteral carrier, by methods known in the art. The injection needs to be essentially sterilized, and be protected from the contamination of microorganisms, such as bacteria and fungi. Examples of the suitable carrier for the injection may include, but are not limited to, water, ethanol, polyols (e.g., glycerol, propylene glycol, liquid polyethylene glycol, etc.), mixtures thereof, and/or solvents or dispersive media containing vegetable oils. More preferably, Hanks' solution, Ringer's solution, phosphate buffered saline (PBS) or sterile water for injection containing triethanolamine, or an isotonic solution (such as 10% ethanol, 40% propylene glycol, or 5% dextrose) may be used as a suitable carrier. In order to protect the injection from microbial contamination, the injection may further contain various antibiotics and antifungal agents, such as paraben, chlorobutanol, phenol sorbic acid, and thimerosal. In most cases, the injection may further contain an isotonic agent, such as sugar or sodium chloride. The form of the transdermal administration preparation includes ointment, cream, lotion, gel, solution for external application, plaster, liniment, and aerosol. The "transdermal administration" means locally administering a pharmaceutical composition to the skin to deliver an effective amount of an active ingredient through the skin. These formulations are described in the literature, which is a formulary generally known in pharmaceutical chemistry (Remington's Pharmaceutical Science, 15th Edition, 1975, Mack Publishing Company, Easton, Pennsylvania).

For other pharmaceutically acceptable carriers, ones disclosed in the following literature may be referred (Remington's Pharmaceutical Sciences, 19th ed., Mack Publishing Company, Easton, PA, 1995).

### Advantageous Effects

As described above, the present invention relates to a method for preparing polymeric microspheres having increased efficiency of drug encapsulation, and more specifically, the present invention provides a method for preparing polymeric microspheres, the method comprising the steps of: preparing an internal water phase (W1) and an oil phase (O); mixing the internal water phase and the oil phase to prepare a W1/O emulsion, followed by cooling; mixing the W1/O emulsion with an external water phase (W2) to prepare a W1/O/W2 emulsion; and drying and recovering the prepared W1/O/W2 emulsion. The method of the present invention can increase the efficiency of drug encapsulation and prepare polymeric microspheres with a high drug content, through the procedure of solidifying the oil phase and the procedure of maintaining the particular temperature for at least a predetermined period of time, and thus the present invention is effective in the preparation of controlled-release drugs.

### Mode for Carrying Out the Invention

Hereinafter, the present invention will be described in detail with reference to the following examples.

However, the following examples are merely for illustrating the present invention and are not intended to limit the scope of the present invention.

### <Comparative examples>

### Test on the efficiency of drug encapsulation in microparticles prepared by conventional methods

### <Comparative Example 1> Measurement of the encapsulation efficiency according to cooling of W1/O emulsion

1 g of distilled water was added to a mixture of 0.45 g of leuprorelin acetate powder and 0.08 g of gelatin, and the powder was dissolved through warming at about 60°C, thereby preparing a W1 (internal water phase) solution. 6.65 g of dichloromethane was added to 4.0 g of a DL-lactic acid-glycolic acid copolymer (weight-average molecular weight: 10,000-15,000) powder, followed by vortexing, thereby preparing an O (oil phase) solution. The W1 solution was added to the O solution, followed by emulsification using a homogenizer, thereby preparing a W1/O emulsion.

The viscosity of the W1/O phase was increased using a chiller maintained at about 3°C, and then the W1/O phase was mixed with an aqueous solution of 1.0 wt% polyvinyl alcohol (PVA) (1250 ml, hereinafter common in volume) adjusted to 25°C, followed by stirring using a homomixer (Siverson, L4R), thereby preparing a W1/O/W2 emulsion. The temperature was continuously maintained at 25°C while the emulsion was prepared. The prepared W1/O/W2 emulsion was dried in water for 4 hours, filtered through a sieve with 75 *µ*m-sized openings, and then centrifuged, thereby recovering microparticles. The recovered microparticles were re-dispersed in distilled water, mixed, and repeatedly centrifuged to sufficiently wash the surface of the microparticles. Thereafter, the obtained polymeric microparticles were freeze-dried to obtain leuprorelin acetate-containing polymeric microspheres (hereinafter, referred to as "polymeric microspheres"). The drug content and the drug encapsulation efficiency of the obtained polymeric microspheres were measured.

Testing for the drug content measurement of the polymeric microspheres was carried out by the following method.

Approximately 50 mg of the polymeric microspheres were precisely weighed and placed into a 100 mL-volume flask, and then the polymeric microspheres were completely dissolved in 2 mL DMSO, and filled to the mark line, followed by ultrasonic extraction, thereby preparing a test liquid. Then, the test liquid was subjected to liquid chromatography to measure the drug content. The liquid chromatography was carried out in the conditions of:
Column: 2. 1 mm X 50 mm, Waters ACQUITY UPLC® CSH C18 1.7 *µ*m
Detector: UV absorptiometer (wavelength 220 nm)
Flow rate: 0.5 mL/min
Mobile phase: 0.2 % Trifluoroacetic acid in Water/Acetonitrile (75/25, v/v).

Test results verified that, in the group tested under the conditions of Comparative Example 1, the content of the drug contained in the dried polymeric microspheres was 3.4% (weight ratio) and the drug encapsulation efficiency was 34.0%.

### <Comparative Example 2> Measurement of the encapsulation efficiency of polymeric microspheres when temperature for preparing W1/O/W2 emulsion was 20°C

0.5 g of distilled water was added to a mixture of 0.50 g of leuprorelin acetate powder and 0.08 g of gelatin, and the powder was dissolved through warming at about 60°C, thereby preparing a W1 solution. 8.0 g of dichloromethane was added to 4.0 g of a DL-lactic acid-glycolic acid copolymer (weight-average molecular weight: 10,000-15,000) powder, followed by vortexing, thereby preparing an O solution. The W1 solution was added to the O solution, followed by emulsification using a homogenizer, thereby preparing a W1/O emulsion.

The viscosity of the W1/O phase was increased using a chiller maintained at about 18°C, and then the W1/O phase was mixed with an aqueous solution of 1.0 wt% polyvinyl alcohol (PVA) adjusted to 20°C, followed by stirring using a homomixer (Siverson, L4R), thereby preparing a W1/O/W2 emulsion. While the emulsion was prepared, the temperature was maintained at 18-20°C, which was lower than that of Comparative Example 1, and the temperature maintained was monitored over time. The temperature maintained is shown in Table 1 below. The prepared W1/O/W2 emulsion was dried in water for 4 hours, filtered through a sieve with 75 *µ*m-sized openings, and then centrifuged, thereby recovering microparticles. The recovered microparticles were re-dispersed in distilled water, mixed, and repeatedly centrifuged to sufficiently wash the surface of the microparticles. Thereafter, the recovered microparticles were freeze-dried to obtain polymeric microspheres. The drug content and the drug encapsulation efficiency of the obtained polymeric microspheres were measured by the same method as in Comparative Example 1.

**[Table 1]**

| Comparision of W1/O/W2 preparation temperature between Comparative Examples 1 and 2 | | | | |
|---|---|---|---|---|
| **W1**/**O**/**W2 emulsion** | **Initial temperature** | **Temperature after 10 min** | **Temperature after 30 min** | **Temperature after 90 min** |
| **Com. Ex. 1** | 25°C | 25°C | 25°C | 25°C |
| **Com. Ex. 2** | 20°C | 18°C | 18°C | 18°C |

Test results verified that, in the group tested under the conditions of Comparative Example 2, the content of the drug contained in the dried polymeric microspheres was 1.6 % (weight ratio) and the drug encapsulation efficiency was 15.0 %.

Thus, it was confirmed that, even when the initial temperature was 20°C and then was maintained at 18°C during the preparation of the W1/O/W2, sufficient drug encapsulation efficiency could not be obtained.

### <Comparative Example 3> Measurement of the encapsulation efficiency of polymeric microspheres when temperature for preparing W1/O/W2 emulsion was 18°C

0.5 g of distilled water was added to a mixture of 0.50 g of leuprorelin acetate powder and 0.08 g of gelatin, and the powder was dissolved through warming at about 60°C, thereby preparing a W1 solution. 8.0 g of dichloromethane was added to 4.0 g of a DL-lactic acid-glycolic acid copolymer (weight-average molecular weight: 10,000-15,000) powder, followed by vortexing, thereby preparing an O solution. The W1 solution was added to the O solution, followed by emulsification using a homogenizer, thereby preparing a W1/O emulsion.

The viscosity of the W1/O phase was increased using a chiller maintained at about 18°C, and then the W1/O phase was mixed with an aqueous solution of 0.25 wt% polyvinyl alcohol (PVA) adjusted to 18°C, followed by stirring using a homomixer (Siverson, L4R), thereby preparing a W1/O/W2 emulsion. While the emulsion was prepared, the temperature was maintained at 18-20°C, which was lower than that of Comparative Example 1, and the temperature maintained over time, starting at 18°C in an initiation stage, was 20°C after a predetermine period of time. The temperature was monitored over time, and the monitored temperature is shown in Table 2 below. The prepared W1/O/W2 emulsion was dried in water for 4 hours, filtered through a sieve with 75 *µ*m-sized openings, and then centrifuged, thereby recovering microparticles. The recovered microparticles were re-dispersed in distilled water, mixed, and repeatedly centrifuged to sufficiently wash the surface of the microparticles. Thereafter, the recovered microparticles were freeze-dried to obtain polymeric microspheres. The drug content and the drug encapsulation efficiency of the obtained polymeric microspheres were measured by the same method as in Comparative Example 1.

**[Table 2]**

| Comparision of W1/O/W2 preparation temperature between Comparative Examples 2 and 3 | | | | |
|---|---|---|---|---|
| **W1/O/W2 emulsion** | **Initial temperature** | **Temperature after 10 min** | **Temperature after 30 min** | **Temperature after 90 min** |
| **Com**. **Ex. 2** | 20°C | 18°C | 18°C | 18°C |
| **Com**. **Ex. 3** | 18°C | 20°C | 20°C | 20°C |

Test results verified that, in the group tested under the conditions of Comparative Example 3, the content of the drug contained was 2.2 % (weight ratio) and the drug encapsulation efficiency was 20.2 %.

Thus, it was confirmed that, even when the initial temperature was 18°C, raised to 20°C within 10 minutes, and then maintained during the preparation of the W1/O/W2, sufficient drug encapsulation efficiency could not be obtained.

### <Examples>

### Test on drug encapsulation efficiency of microparticles prepared by the method of present invention

### <Example 1> Measurement of encapsulation efficiency of polymeric microspheres when temperature for preparing W1/O/W2 emulsion was maintained at 18°C for 10 minutes or longer

0.5 g of distilled water was added to a mixture of 0.50 g of leuprorelin acetate powder and 0.08 g of gelatin, and the powder was dissolved through warming at about 60°C, thereby preparing a W1 (internal water phase) solution. 8.0 g of dichloromethane was added to 4.0 g of a DL-lactic acid-glycolic acid copolymer (weight-average molecular weight: 10,000-15,000) powder, followed by vortexing, thereby preparing an O solution. The W1 solution was added to the O solution, followed by emulsification using a homogenizer, thereby preparing a W1/O emulsion.

The viscosity of the W1/O phase was increased using a chiller maintained at about 18°C, and then the W1/O phase was mixed with an aqueous solution of 0.25 wt% polyvinyl alcohol (PVA) adjusted to 18°C, followed by stirring using a homomixer (Siverson, L4R), thereby preparing a W1/O/W2 emulsion. While the emulsion was prepared, the temperature, starting at 18°C, was maintained at the same temperature for 10 minutes, then gently raised to 23°C for 60 minutes. The temperature was monitored over time, and the monitored temperature is shown in Table 3 below. The prepared W1/O/W2 emulsion was dried in water for 4 hours, filtered through a sieve with 75 *µ*m-sized openings, and then centrifuged, thereby recovering microparticles. The recovered microparticles were re-dispersed in distilled water, mixed, and repeatedly centrifuged to sufficiently wash the surface of the microparticles. Thereafter, the recovered microparticles were freeze-dried to obtain polymeric microspheres. The drug content and the drug encapsulation efficiency of the obtained polymeric microspheres were measured by the same method as in Comparative Example 1.

**[Table 3]**

| Comparision of W1/O/W2 preparation temperature between Comparative Example 3 and Example 1 | | | | |
|---|---|---|---|---|
| **W1/O/W2 emulsion** | **Initial temperature** | **Temperature after 10 min** | **Temperature after 30 min** | **Temperature after 90 min** |
| **Com. Ex. 3** | 18°C | 20°C | 20°C | 20°C |
| **Ex. 1** | 18°C | 18°C | 21°C | 23°C |

Test results verified that, in the group tested under the conditions of Example 1, the content of the drug contained was 8.1% (weight ratio) and the drug encapsulation efficiency was 74.0 %.

Thus, it was verified that, compared with Comparative Example 3 in which the initial temperature was identical but the temperature was raised to 20 within 10 minutes, in Example 1 where the temperature was maintained at 18°C for up to 10 minutes, the drug content and the drug encapsulation efficiency was significantly increased to 366% compared with comparative example 3. Thus, it was confirmed that, when the W2 formation temperature began at 18°C or lower and the temperature was maintained for 10 minutes or more for the preparation of W1/O/W2 type polymeric microspheres, the drug content and the drug encapsulation efficiency were significantly improved.

### <Example 2> Measurement of encapsulation efficiency of polymeric microspheres when temperature for preparing W1/O/W2 emulsion was maintained at 15 °C for 30 minutes or longer

1.0 g of distilled water was added to a mixture of 0.52 g of leuprorelin acetate powder and 0.09 g of gelatin, and the powder was dissolved through warming at about 60 °C, thereby preparing a W1 (internal water phase) solution. 13.3 g of dichloromethane was added to 4.0 g of a DL-lactic acid-glycolic acid copolymer (weight-average molecular weight: 10,000-15,000) powder, followed by vortexing, thereby preparing an O solution. The W1 solution was added to the O solution, followed by emulsification using a homogenizer, thereby preparing a W1/O emulsion.

The viscosity of the W1/O phase was increased using a chiller maintained at about 15 °C, and then the W1/O phase was mixed with an aqueous solution of 0.25 wt% polyvinyl alcohol (PVA) adjusted to 15 °C, followed by stirring using a homomixer (Siverson, L4R), thereby preparing a W1/O/W2 emulsion (rotation number: about 5,000 rpm). While the emulsion was prepared, the temperature was maintained at 15-20 °C, which was lower than that in Comparative Example 4, and the period of time for maintaining the initial low temperature was increased. The temperature was monitored over time, and the monitored temperature is shown in Table 4 below. The prepared W1/O/W2 emulsion was dried in water for 4 hours, filtered through a sieve with 75 *µ*m-sized openings, and then centrifuged, thereby recovering microparticles. The recovered microparticles were re-dispersed in distilled water, mixed, and repeatedly centrifuged to sufficiently wash the surface of the microparticles. Thereafter, the recovered microparticles were freeze-dried to obtain polymeric microspheres. The drug content and the drug encapsulation efficiency of the obtained polymeric microspheres were measured by the same method as in Comparative Example 1.

**[Table 4]**

| Comparision of W1/O/W2 preparation temperature between Examples 1 and 2 | | | | | | |
|---|---|---|---|---|---|---|
| **W1/O/W2 emulsion** | **Initial temp** | **Temp after 10 min** | **Temp after 30 min** | **Temp after 60 min** | Temp **after 70 min** | **Temp after 90 min** |
| **Ex. 1** | 18°C | 18°C | 21°C | 23°C | | |
| **Ex. 2** | 15°C | 15°C | 15°C | | 18°C | 20°C |

Test results verified that, in the group tested under the conditions of Example 2, the content of the drug contained was 10.8% (weight ratio) and the drug encapsulation efficiency was 96%.

Thus, it was confirmed that, when the period of time for maintaining a critical temperature of W2 was increased to 30 minutes, the drug encapsulation efficiency was more improved.

### <Examples 3 and 4> Measurement of polymeric microspheres when temperature for preparing W1/O/W2 emulsion was continuously maintained at 18°C or lower

0.5 g of distilled water was added to a mixture of 0.50 g of leuprorelin acetate powder and 0.08 g of gelatin, and the powder was dissolved through warming at about 60°C, thereby preparing a W1 (internal water phase) solution. 8.0 g of dichloromethane was added to 4.0 g of a DL-lactic acid-glycolic acid copolymer (weight-average molecular weight: 10,000-15,000) powder, followed by vortexing, thereby preparing an O solution. The W1 solution was added to the O solution, followed by emulsification using a homogenizer, thereby preparing a W1/O emulsion.

The viscosity of the W1/O phase was increased using a chiller maintained at 18°C (Example 3) or 17°C (Example 4), and then the W1/O phase was mixed with an aqueous solution of 0.25 wt% polyvinyl alcohol (PVA) adjusted to 18°C or 17°C, followed by stirring using a homomixer (Siverson, L4R), thereby preparing a W1/O/W2 emulsion. While the emulsion was prepared, the temperature was maintained at 18°C or lower, and the temperature was monitored over time. The temperature maintained is shown in Table 5. The prepared W1/O/W2 emulsion was dried in water for 4 hours, filtered through a sieve with 75 *µ*m-sized openings, and then centrifuged, thereby recovering microparticles. The recovered microparticles were re-dispersed in distilled water, mixed, and repeatedly centrifuged to sufficiently wash the surface of the microparticles. Thereafter, the recovered microparticles were freeze-dried to obtain polymeric microspheres. The drug content and the drug encapsulation efficiency of the obtained polymeric microspheres were measured by the same method as in Comparative Example 1.

**[Table 5]**

| Comparision of W1/O/W2 preparation temperature among Examples 2 to 4 | | | | | |
|---|---|---|---|---|---|
| W1/O/We Emulsion | Initial Temp | Temp after 10 min | Temp after 30 min | Temp after 70 min | Temp after 90 min |
| Ex. 2 | 15 °C | 15 °C | 15 °C | 18 °C | 20 °C |
| Ex. 3 | 18 °C | 18 °C | 18 °C | 18 °C | 18 °C |
| Ex. 4 | 17 °C | 17 °C | 18 °C | 18 °C | 18 °C |

Test results verified that, in the group tested under the conditions of Example 3, the content of the drug contained was 10.3% (weight ratio) and the drug encapsulation efficiency was 94 %, while, in the group tested under the conditions of Example 4, the content of the drug contained was 10.4% (weight ratio) and the drug encapsulation efficiency was 95%.

Thus, the maintenance of W2 at 15 °C - 18 °C greatly improved the drug encapsulation efficiency compared with conventional preparation methods, thereby preparing polymeric microspheres through successful drug encapsulation.

### Industrial Applicability

As set forth above, the present invention relates to a method for preparing polymeric microspheres having increased drug encapsulation efficiency, and more specifically, the present invention provides a method for preparing polymeric microspheres, the method comprising the steps of: preparing an internal water phase (W1) and an oil phase (O); mixing the internal water phase and the oil phase to prepare a W1/O emulsion, followed by cooling; mixing the W1/O emulsion with an external water phase (W2) to prepare a W1/O/W2 emulsion; and drying and recovering the prepared W1/O/W2 emulsion. The method of the present invention can increase the drug encapsulation efficiency and prepare polymeric microspheres with a high drug content, through the procedure of solidifying the oil phase and the procedure of maintaining the particular temperature for at least a predetermined period of time. Therefore, the present invention is effective in the preparation of controlled-release drugs, and thus is highly industrially applicable.

## Claims

1. A method for preparing polymeric microspheres, the method comprising the steps of:
(a) preparing an internal water phase (W1) containing a drug, a thickener, and an aqueous solvent, and preparing an oil phase (O) containing a polymer and a fat-soluble solvent;
(b) mixing the internal water phase and the oil phase prepared in step (a) to prepare a W1/O emulsion, and cooling the prepared W1/O emulsion to increase viscosity of the W1/O emulsion;
(c) dispersing the W1/O emulsion, of which the viscosity is increased in step (b), in an external water phase (W2), which contains a water-soluble solution and of which the temperature is maintained at 18°C or lower, followed by mixing while the temperature is maintained at 18°C or lower for 10 minutes or more, thereby preparing a W1/O/W2 emulsion; and
(d) drying and recovering the W1/O/W2 emulsion prepared in step (c).

2. The method of claim 1, wherein the temperature of the external water phase (W2) in step (c) is in a range of 10°C to 18 °C.

3. The method of claim 1, wherein the temperature for dispersing and mixing the W1/O emulsion with increased viscosity in the external water phase (W2) in step (c) is in a range of 10 °C to 18 °C.

4. The method of claim 1, wherein the period of time for maintaining the temperature at which the W1/O emulsion with increased viscosity is dispersed and mixed in the external water phase (W2) in step (c) is 10 minutes or longer.

5. The method of claim 3, wherein the temperature is in a range of 15°C to 18°C.

6. The method of claim 4, wherein the period of time is 30 minutes or longer.

7. The method of claim 1, wherein the drug is leuprorelin acetate.

8. Polymeric microspheres prepared by the method of claim 1.
